# EUROPEAN PATENT SPECIFICATION

(11) **EP 2 075 311 B1**
(45) Date of publication and mention of the grant of the patent: **14.12.2011**
(21) Application number: 07829742.1
(22) Date of filing: 12.10.2007
(51) Int. Cl.: C09K 11/06, C08F 12/26, C08F 12/32, H01L 51/50, H01L 51/00, H05B 33/14

(54) **ORGANIC LIGHT-EMITTING DEVICE**
ORGANISCHES LEUCHTBAUELEMENT
DISPOSITIF ÉLECTROLUMINESCENT ORGANIQUE

(30) Priority: 16.10.2006 JP 2006281557
(43) Date of publication of application: 01.07.2009
(73) Proprietor: Showa Denko K.K., Tokyo 105-8518 (JP)
(72) Inventor: TOBA, Masahiko, Chiba-shi Chiba 267-0056 (JP)
(74) Representative: Strehl Schübel-Hopf & Partner
(86) International application number: PCT/JP2007/070006
(87) International publication number: WO 2008/047730

(56) References cited:
- WO-A2-2005/059951
- JP-A- 2004 185 967
- JP-A- 2005 097 589
- JP-A- 2007 063 317
- JP-A- 2007 257 897
- TSUYOSHI MICHINOBU, JUN INUI, HIROYUKI NISHIDE: "Preparation of non-Kekulé- and nondisjoint-type aromatic polyamines by palladium-catalyzed polycondensation and their poly(cationic radical)s" POLYHEDRON, vol. 22, no. 14-17, 15 July 2003 (2003-07-15), pages 1945-1949, XP002572234 Elsevier Science Ltd ISSN: 0277-5387 DOI: 10.1016/S0277-5387(03)00170-0

## Description

### FIELD OF THE INVENTION

The present invention relates to organic light-emitting devices. In more detail, the invention relates to organic light-emitting devices that have at least one organic layer sandwiched between anode and cathode.

### BACKGROUND OF THE INVENTION

Multilayer organic light-emitting devices in which a light-emitting layer of a phosphorescent low-molecular weight compound is provided between a hole transport layer and an electron transport layer are known in the art. The hole transport layer is made of a low-molecular weight compound such as a triphenylamine derivative (Patent Document 1).

These layers are generally formed by vacuum deposition of low-molecular weight compounds. However, vacuum deposition entails a vacuum apparatus, tends to form layers in nonuniform thickness, and causes difficulty in forming layers with a large area. Further, organic light-emitting devices having a luminescent layer of phosphorescent low-molecular weight compound have poor durability.

Meanwhile, phosphorescent polymer compounds obtained by copolymerizing a phosphorescent polymerizable compound and a charge transport polymerizable compound have been developed. The phosphorescent polymer compounds can form a light-emitting layer by simple methods such as spin coating. For example, Patent Document 2 discloses copolymers of triphenylamine derivative and iridium complex.
Patent Document 1: JP-A-2003-308978
Patent Document 2: JP-A-2005-97589 JP 2004-185967 discloses organic electroluminescence devices having an organic layer that comprises a phosphorescent compound and a polymer compound including hole transport structural units and electron transport structural units.

### DISCLOSURE OF THE INVENTION

The copolymers described in Patent Document 2 provide high luminous efficiency but are still insufficient in maximum attainable brightness and life duration.

It is therefore an object of the present invention to provide organic light-emitting devices having high brightness and long life as well as high luminous efficiency.

The present inventors studied diligently to achieve the above object. They have then found that organic light-emitting devices having high brightness and long life as well as high luminous efficiency are obtained by the use of polymer compounds that include structural units derived from a specific hole transport polymerizable compound. The present invention has been completed based on the finding.

The present invention is summarized as follows.

[1] An organic light-emitting device that includes at least one organic layer sandwiched between anode and cathode wherein at least one of the at least one organic layer is a light-emitting layer that comprises a polymer compound including structural units from a polymerizable compound (A) of Formula (1) below: 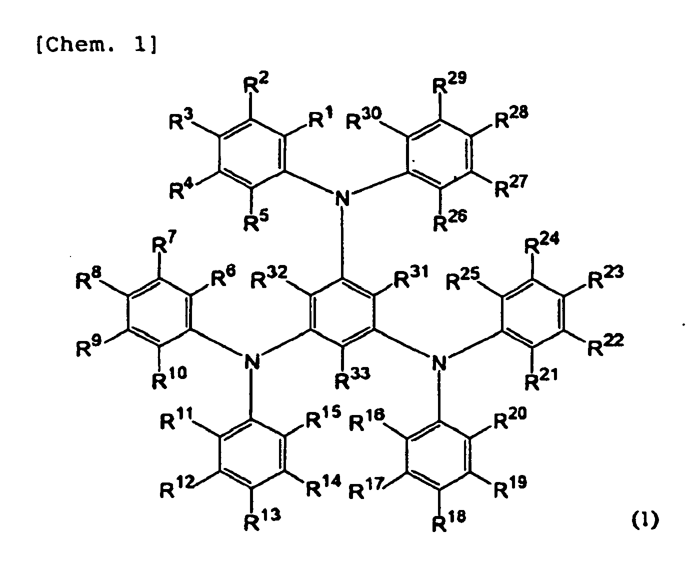 wherein R²⁹ is a substituent group having an alkenyl group, R⁴, R⁹, R¹⁴, R¹⁹ and R²² are an atom or a substituent group selected from the group consisting of halogen atoms, a cyano group, C1-10 alkyl groups, C6-10 aryl groups, amino groups optionally substituted with a C1-10 alkyl group, C1-10 alkoxy groups and silyl groups, and the substituent groups other than R²⁹, R⁴, R⁹, R¹⁴ , R¹⁹ and R²² are hydrogen atom.
[2] The organic light-emitting device described in [1], wherein the polymer compound further includes structural units from a phosphorescent polymerizable compound (B).

[3] The organic light-emitting device described in [1]_{,} wherein the polymer compound further includes structural units from a phosphorescent polymerizable compound (B) and structural units from an electron transport polymerizable compound (C).

[4] The organic light-emitting device described in [2] or [3], wherein the phosphorescent polymerizable compound (B) is a complex represented by Formula (2-1) below:

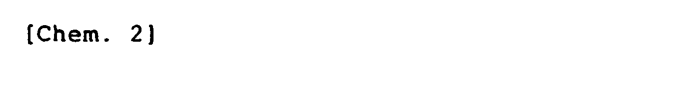

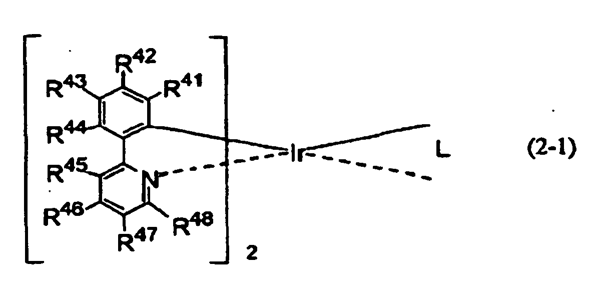

wherein R⁴¹ to R⁴⁸ are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; a pair of R⁴¹ and R⁴² , a pair of R⁴² and R⁴³, a pair of R⁴³ and R⁴⁴ , a pair of R⁴⁴ and R⁴⁵, a pair of R⁴⁵ and R⁴⁶, a pair of R⁴⁶ and R⁴⁷, or a pair of R⁴⁷ and R⁴⁸ may be linked together to form a condensed ring; and L is a bidentate ligand selected from Formulae (2-2) to (2-4) below:

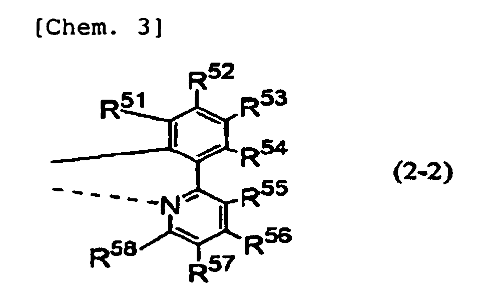

wherein at least one of R⁵¹ to R⁵⁸ is a substituent group having a polymerizable functional group, and those of R⁵¹ to R⁵⁸ that are not substituent groups having a polymerizable functional group are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; and a pair of R⁵¹ and R⁵², a pair of R⁵² and R⁵³, a pair of R⁵³ and R⁵⁴, a pair of R⁵⁴ and R⁵⁵, a pair of R⁵⁵ and R⁵⁶, a pair of R⁵⁶ and R⁵⁷, or a pair of R⁵⁷ and R⁵⁸ may be linked together to form a condensed ring;

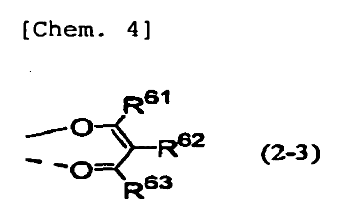

wherein at least one of R⁶¹ to R⁶³ is a substituent group having a polymerizable functional group, and those of R⁶¹ to R⁶³ that are not substituent groups having a polymerizable functional group are each independently an atom or a substituent group selected from the group consisting of a hydrogen atom, a cyano group, C1-10 alkyl groups, C6-10 aryl groups, amino groups optionally substituted with a C1-10 alkyl group, C1-10 alkoxy groups and silyl groups; and a pair of R⁶¹ and R⁶² or a pair of R⁶² and R⁶³ may be linked together to form a condensed ring;

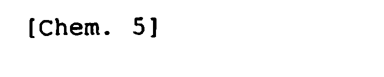

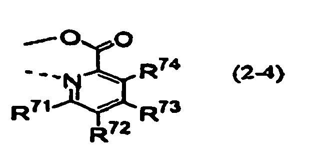

wherein at least one of R⁷¹ to R⁷⁴ is a substituent group having a polymerizable functional group, and those of R⁷¹ to R⁷⁴ that are not substituent groups having a polymerizable functional group are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; and a pair of R⁷¹ and R⁷², a pair of R⁷² and R⁷³, or a pair of R⁷³ and R⁷⁴ may be linked together to form a condensed ring.
[5] The organic light-emitting device described in [1], wherein the light-emitting layer further comprises a phosphorescent compound.

[6] The organic light-emitting device described in [1], wherein the light-emitting layer further comprises a phosphorescent compound and the polymer compound further includes structural units from an electron transport polymerizable compound (C).

[7] The organic light-emitting device described in [3] or [6], wherein the electron transport polymerizable compound (C) is an oxadiazole derivative or a triarylborane derivative.

[8] A surface-emitting light source wherein the organic light-emitting device described in any one of [1] to [7] is used.

[9] An image display wherein the organic light-emitting device described in any one of [1] to [7] is used.

### ADVANTAGES OF THE INVENTION

The organic light-emitting devices according to the present invention have high brightness and long life as well as high luminous efficiency.

### BRIEF DESCRIPTION OF THE DRAWING

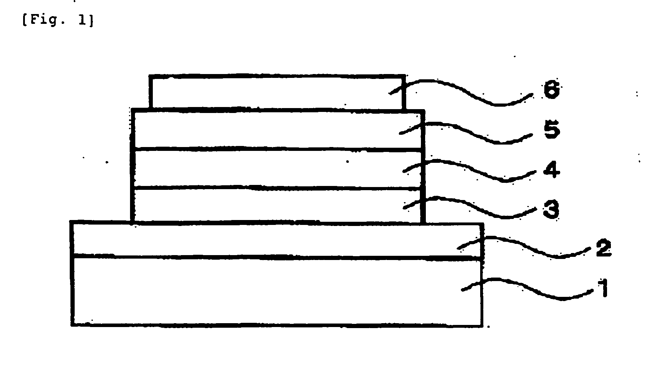

Fig. 1 is a cross sectional view of an organic light-emitting device according to an embodiment of the invention.

### DESCRIPTION OF NUMERALS

1: glass substrate
2: anode
3: hole transport layer
4: light-emitting layer
5: electron transport layer
6: cathode

### PREFERRED EMBODIMENTS OF THE INVENTION

The present invention will be described in detail hereinbelow.

### 1. Light-emitting layer

An organic light-emitting device of the present invention has at least one organic layer sandwiched between anode and cathode, and at least one of the at least one organic layer is a light-emitting layer that comprises a specific polymer compound. The polymer compound is a compound including structural units from a hole transport polymerizable compound (A) of Formula (1) as described hereinabove. The polymer compound may further include structural units from a phosphorescent polymerizable compound (B) and/or structural units from an electron transport polymerizable compound (C). The polymer compound may be obtained by polymerizing a hole transport polymerizable compound (A) optionally together with a phosphorescent polymerizable compound (B) and/or an electron transport polymerizable compound (C).

In detail, the polymer compounds may be: polymer compounds (I) which are obtained by polymerizing a polymerizable compound (A) of Formula (1) and a phosphorescent polymerizable compound (B) and which include structural units from the hole transport polymerizable compound (A) and structural units from the phosphorescent polymerizable compound (B);
polymer compounds (II) which are obtained by polymerizing a polymerizable compound (A) of Formula (1), a phosphorescent polymerizable compound (B) and an electron transport polymerizable compound (C) and which include structural units from the hole transport polymerizable compound (A), structural units from the phosphorescent polymerizable compound (B) and structural units from the electron transport polymerizable compound (C);
polymer compounds (III) which are obtained by polymerizing a polymerizable compound (A) of Formula (1) and which include structural units from the hole transport polymerizable compound (A); or
polymer compounds (IV) which are obtained by polymerizing a polymerizable compound (A) of Formula (1) and an electron transport polymerizable compound (C) and which include structural units from the hole transport polymerizable compound (A) and structural units from the electron transport polymerizable compound (C).

When the polymer compounds include structural units from the compound (A) and structural units from the phosphorescent polymerizable compound (B) (e.g., the polymer compounds (I) and (II)), such polymer compounds alone can form a light-emitting layer.

When the polymer compounds include structural units from the compound (A) but do not contain structural units from the phosphorescent polymerizable compound (B) (e.g., the polymer compounds (III) and (IV)), such polymer compounds together with a phosphorescent low-molecular weight compound form a light-emitting layer. In detail, the light-emitting layer in this case contains the polymer compound (III) and a phosphorescent low-molecular weight compound, or the polymer compound (IV) and a phosphorescent low-molecular weight compound.

The polymer compounds having structural units from the compound (A) such as the polymer compounds (I) to (IV) possess excellent hole transport properties whereby organic light-emitting devices having high luminous efficiency and high brightness may be obtained. Further, the polymer compounds have a high glass transition temperature and excellent heat resistance whereby the life of organic light-emitting devices may be extended.

In the specification, hole transport polymerizable compounds and electron transport polymerizable compounds may be collectively referred to as "carried transport polymerizable compounds".

In the compounds (A) of Formula (1), R²⁹ is a substituent group having a polymerizable functional group.

Such substituent groups are not particularly limited as long as having a polymerizable functional group. Specific examples of the substituent groups having a polymerizable functional group include C1-10 alkyl groups, C6-10 aryl groups, amino groups optionally substituted with a C1-10 alkyl group, C1-10 alkoxy groups and silyl groups as will be described later.

The polymerizable functional group is an alkenyl group.

In a preferred embodiment of the compounds (A), the alkenyl group is present in a substituent group illustrated in Formulae (a1) to (a12) below. In particular, substituent groups represented by Formulae (a1), (a5), (a8) and (a12) are more preferable because such functional groups may be easily introduced into the compounds.

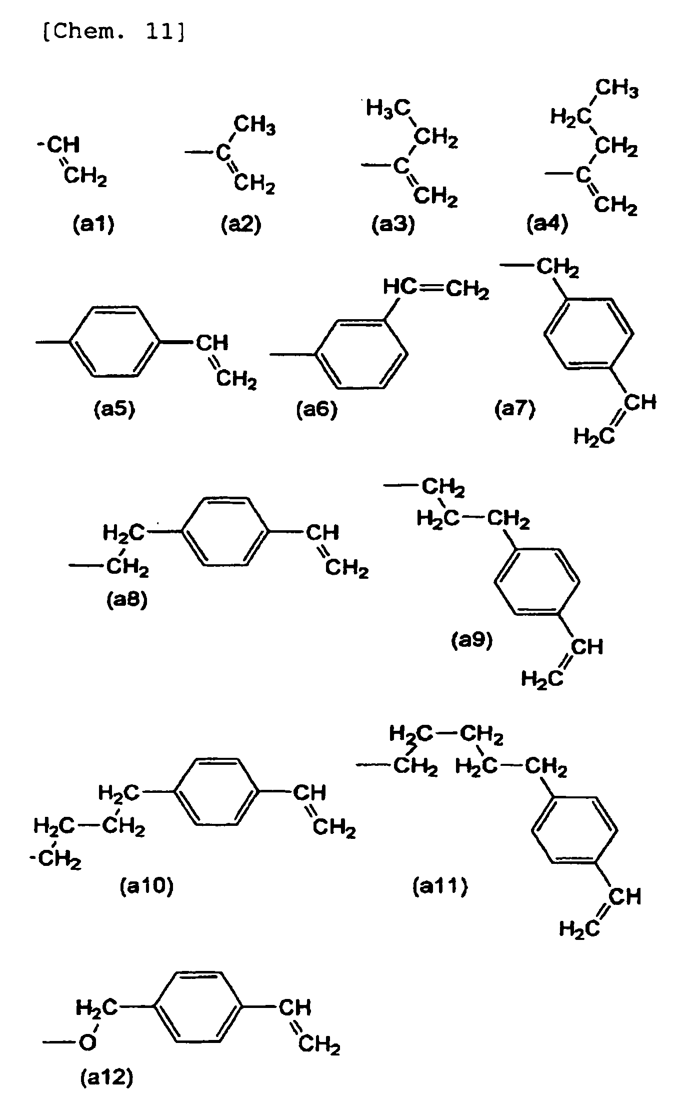

R⁴, R⁹, R¹⁴, R¹⁴, R¹⁹ and R²² are an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups.

Examples of the halogen atoms include fluorine, chlorine, bromine and iodine atoms.

Examples of the C1-10 alkyl groups include methyl, ethyl, propyl, isopropyl, butyl, isobutyl, t-butyl, amyl, hexyl, octyl and decyl groups.

Examples of the C6-10 aryl groups include phenyl, tolyl, xylyl, mesityl and naphthyl groups.

Examples of the amino groups optionally substituted with a C1-10 alkyl group include amino, dimethylamino, diethylamino and dibutylamino groups.

Examples of the C1-10 alkoxy groups include methoxy, ethoxy, propoxy, isopropoxy, butoxy, isobutoxy, t-butoxy, hexyloxy, 2-ethylhexyloxy and decyloxy groups.

Examples of the silyl groups include trimethylsilyl, triethylsilyl, t-butyldimethylsilyl and trimethoxysilyl groups.

In view of life and luminous efficiency of organic light-emitting devices, compound (A) is a compound in which R²⁹ is the substituent group having a polymerizable functional group, R⁴, R⁹, R¹⁴, R¹⁹ and R²² are an atom or a substituent group selected from the group consisting of halogen atoms, a cyano group, C1-10 alkyl groups, C6-10 aryl groups, amino groups optionally substituted with a C1-10 alkyl group, C1-10 alkoxy groups and silyl groups, and the substituent groups other than R²⁹, R⁴, R⁹, R¹⁴ , R¹⁹ and R²² are hydrogen.

In the respective sets of substituent groups R¹ to R⁵, R⁶ to R¹⁰, R¹¹ to R¹⁵, R¹⁶ to R²⁰, R²¹ to R²⁵, and R²⁶ to R³⁰, any two substituent groups bonded to adjacent carbon atoms on the benzene ring may be linked together to form a condensed ring.

The compounds (A) may be used singly, or two or more kinds may be used in combination.

The compounds (A) may be produced by palladium-catalyzed substitution reaction of 1,3, 5-triaminobenzene and aryl halide, or diarylamine and 1, 3, 5-trihalogenobenzene. The substitution reaction is described in detail in for example Tetrahedron Letters, 1998, Vol. 39, p. 2367.

The compounds (B) in the invention are low-molecular weight compounds which contain a substituent group having a polymerizable functional group and can emit light from the triplet excited state at room temperature. Such compounds may be used without limitation in the invention. Preferred examples include palladium complexes, osmium complexes, iridium complexes, platinum complexes and gold complexes each having a substituent group having a polymerizable functional group. Iridium complexes and platinum complexes are more preferable, and iridium complexes are most preferable. The substituent groups having a polymerizable functional group have the same definition and preferred embodiments as those of the substituent groups having a polymerizable functional group in the compounds (A). The compounds (B) may be used singly, or two or more kinds may be used in combination.

Of the iridium complexes, complexes represented by Formula (2-1) described hereinabove may be suitably used.

In Formula (2-1), R⁴¹ to R⁴⁸ are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups. Examples of these atoms and substituent groups are as described hereinabove.

Preferably, R⁴¹ to R⁴⁸ are each independently a hydrogen atom, a fluorine atom, a cyano group, a methyl group, a t-butyl group, a dimethylamino group, a butoxy group or a 2-ethylhexyloxy group. More preferably, R⁴² is a t-butyl group and R⁴¹ to R⁴⁸ except R⁴² are each a hydrogen atom.

A pair of R⁴¹ and R⁴², a pair of R⁴² and R⁴³, a pair of R⁴³ and R⁴⁴ , a pair of R⁴⁴ and R⁴⁵, a pair of R⁴⁵ and R⁴⁶, a pair of R⁴⁶ and R⁴⁷, or a pair of R⁴⁷ and R⁴⁸ may be linked together to form a condensed ring.

The letter L is a bidentate ligand selected from Formulae (2-2) to (2-4) hereinabove.

In Formula (2-2), at least one of R⁵¹ to R⁵⁸ is a substituent group having a polymerizable functional group. The substituent groups having a polymerizable functional group have the same definition and preferred embodiments as those of the substituent groups having a polymerizable functional group in the compounds (A). Preferably, R⁵² is the substituent group having a polymerizable functional group.

Of R⁵¹ to R⁵⁸, those that are not the substituent groups having a polymerizable functional group are each independently an atom or a substituent group as described for R⁴¹. Preferably, R⁵¹ to R⁵⁸ are each independently a hydrogen atom, a fluorine atom, a cyano group, a methyl group, a t-butyl group, a dimethylamino group, a butoxy group or a 2-ethylhexyloxy group.

A pair of R⁵¹ and R⁵², a pair of R⁵² and R⁵³, a pair of R⁵³ and R⁵⁴, a pair of R⁵⁴ and R⁵⁵, a pair of R⁵⁵ and R⁵⁶, a pair of R⁵⁶ and R⁵⁷, or a pair of R⁵⁷ and R⁵⁸ may be linked together to form a condensed ring.

In Formula (2-3), at least one of R⁶¹ to R⁶³ is a substituent group having a polymerizable functional group. The substituent groups having a polymerizable functional group have the same definition and preferred embodiments as those of the substituent groups having a polymerizable functional group in the compounds (A). Preferably, R⁶³ is the substituent group having a polymerizable functional group.

Of R⁶¹ to R⁶³, those that are not the substituent groups having a polymerizable functional group are each independently an atom or a substituent group as described for R⁴¹ (except for halogen atoms). Preferably, R⁶¹ to R⁶³ are each independently a methyl group, a t-butyl group, a dimethylamino group or a methoxy group.

A pair of R⁶¹ and R⁶² or a pair of R⁶² and R⁶³ may be linked together to form a condensed ring.

In Formula (2-4), at least one of R⁷¹ to R⁷⁴ is a substituent group having a polymerizable functional group. The substituent groups having a polymerizable functional group have the same definition and preferred embodiments as those of the substituent groups having a polymerizable functional group in the compounds (A). Preferably, R⁷² is the substituent group having a polymerizable functional group.

Of R⁷¹ to R⁷⁴, those that are not the substituent groups having a polymerizable functional group are each independently an atom or a substituent group as described for R⁴¹. Preferably, R⁷¹ to R⁷⁴ are each independently a hydrogen atom, a fluorine atom, a cyano group, a methyl group, a t-butyl group, a dimethylamino group, a butoxy group or a 2-ethylhexyloxy group.

A pair of R⁷¹ and R⁷², a pair of R⁷² and R⁷³, or a pair of R⁷³ and R⁷⁴ may be linked together to form a condensed ring.

The iridium complex of Formula (2-1) may be produced for example as follows. A specific bidentate ligand and an iridium compound such as iridium chloride (0.5 equivalent weight) are reacted with each other in a solvent such as 2-ethoxyethanol. The resultant metal complex and a bidentate ligand having a polymerizable functional group are heated in a solvent such as 2-ethoxyethanol together with sodium carbonate. The product is purified, and an iridium complex represented by Formula (2-1) is obtained. The bidentate ligand having a polymerizable functional group may be obtained by a known method.

The compounds (C) for use in the invention are known electron transport compounds that have a substituent group having a polymerizable functional group. Such compounds may be used without limitation. The substituent groups having a polymerizable functional group have the same definition and preferred embodiments as those of the substituent groups having a polymerizable functional group in the compounds (A). In particular, oxadiazole derivatives and triarylborane derivatives are suitably used. The compounds (C) may be used singly, or two or more kinds may be used in combination.

The compounds (C) may be produced by known methods.

Production of the polymer compounds may involve other polymerizable compounds. Such additional polymerizable compounds are not particularly limited and may be compounds without carrier transport properties with examples including alkyl (meth)acrylates such as methyl acrylate and methyl methacrylate, styrene and derivatives thereof. The content of structural units from such additional polymerizable compounds in the polymer compounds is preferably 0 to 50 mol%.

The polymer compounds may be produced by radical polymerization, cationic polymerization, anionic polymerization or addition polymerization, and preferably by radical polymerization.

The glass transition temperature of the polymer compounds is preferably 100 to 250°C, more preferably 150 to 200°C, and still more preferably 175 to 200°C. The glass transition temperature in this range ensures that material deterioration during the manufacturing or driving of the organic light-emitting devices is unlikely and the life extension can be expected.

Low-molecular weight compounds achieve a glass transition temperature of about 100 to 150°C by introduction of bulky substituent groups such as t-butyl group or rigid substituent groups such as naphthyl group. In the polymer compounds contained in the device of the present invention, triphenylamine oligomerization, that is, triphenylamine polymerization in addition to the starburst molecular skeleton allow for a high glass transition temperature.

Desirably, the polymer compounds have a weight average molecular weight of 1,000 to 2,000,000, and preferably 5,000 to 1, 000, 000. This weight average molecular weight ensures that the polymer compound dissolves in organic solvents and forms a uniform thin film. The weight average molecular weight is measured by gel permeation chromatography (GPC) in tetrahydrofuran solvent at 40°C.

The polymer compounds (I) and (II) preferably have an m/ (m + n) ratio (m and n are integers of 1 or greater) of 0.001 to 0.5, and more preferably 0.001 to 0.2 wherein the ratio indicates the proportion in number of structural units derived from the compound (B) relative to all the structural units and further wherein m is the number of structural units from the compound (B) and n is the number of structural units from the carrier transport polymerizable compound(s) (i.e., the number of structural units from the compound (A) in the case of the polymer compounds (I), and the total number of structural units from the compounds (A) and (C) in the case of the polymer compounds (II)). The m/(m + n) ratio in the above range ensures that the obtainable organic light-emitting devices have high carrier mobility, low concentration quenching effect and high luminous efficiency.

In the polymer compounds (II) and (IV), the number n described above satisfies the relation n = x + y wherein x is the number of structural units from the compound (A) and y is the number of structural units from the compound (C) (x and y are integers of 1 or greater). Optimum proportions of structural units derived from the compound (A) and from the compound (C) relative to the structural units from the carrier transport compounds, represented by x/n and y/n respectively, are determined depending on charge transport capability and concentrations of the respective structural units. When the light-emitting layer of the organic light-emitting device is formed from the polymer compound (II) alone, x/n and y/n are each preferably in the range of 0.05 to 0. 95, and more preferably 0.20 to 0.80. When the light-emitting layer is formed from the polymer compound (IV) and a phosphorescent low-molecular weight compound, x/n and y/n are each preferably in the range of 0.05 to 0.95, and more preferably 0.20 to 0.80. Herein, x/n + y/n = 1. The proportions of the respective structural units in the polymer compounds are estimated by ICP elemental analysis and ¹³C-NMR.

By appropriately polymerizing the compound (A) optionally together with the compounds (B) and (C) in proportions controlled in the above-mentioned ranges, the desired polymer compound may be obtained.

The polymer compounds may be random copolymers, block copolymers or alternate copolymers.

When the light-emitting layer is formed from the polymer compound (I) obtained by polymerizing the compounds (A) and (B), a separate electron transport layer may be provided. However, it is preferable to form the light-emitting layer from the polymer compound (I) and an electron transport compound. A single or a mixture of two or more electron transport compounds may be used. The electron transport compounds may be known compounds as will be described later, and oxadiazole derivatives and triarylborane derivatives are preferably used. In this case, the light-emitting layer preferably contains the electron transport compound at 5 to 95 parts by weight, and more preferably 20 to 80 parts by weight based on 100 parts by weight of the polymer compound (I).

When the light-emitting layer is formed from the polymer compound (II) obtained by polymerizing the compounds (A), (B) and (C), the polymer compound alone can form the light-emitting layer.

When the light-emitting layer is formed from the polymer compound (III), the polymer compound (III) and a phosphorescent low-molecular weight compound are used. A single or a mixture of two or more phosphorescent low-molecular weight compounds may be used. Further, a separate electron transport layer may be provided. Still further, the light-emitting layer may be formed from the polymer compound (III), a phosphorescent low-molecular weight compound and an electron transport compound. The phosphorescent low-molecular weight compounds used herein may be known compounds, and iridium complexes may be suitably used. Specific examples thereof include complexes (E-1) to (E-39) illustrated below:

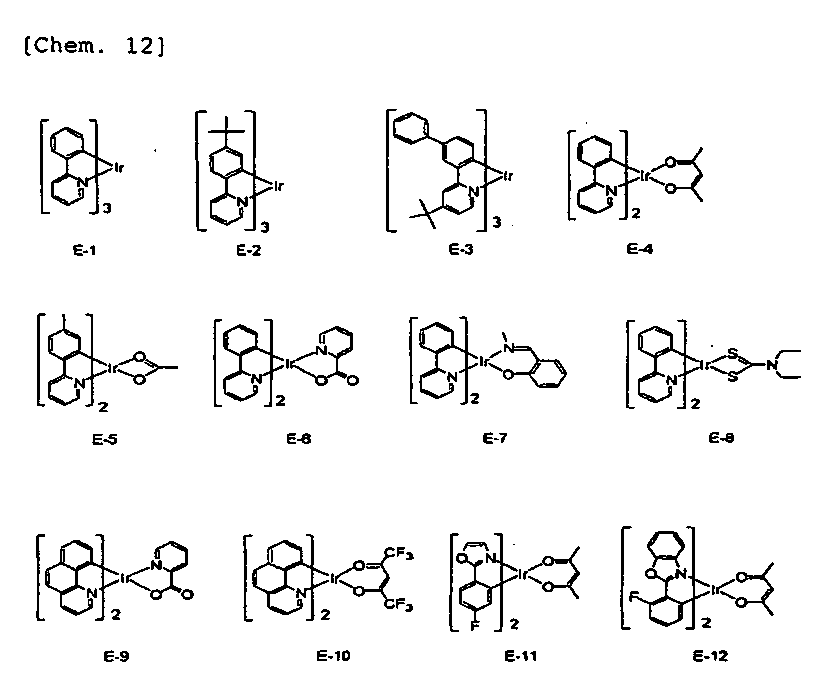

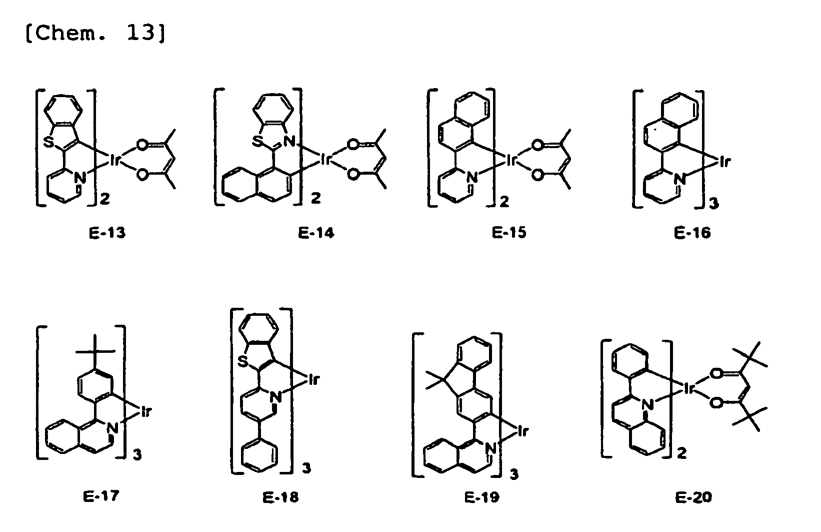

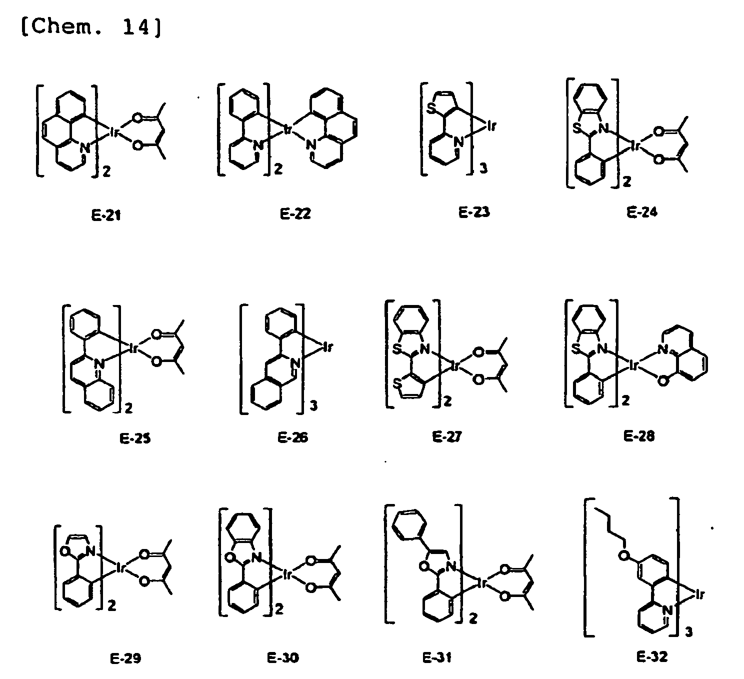

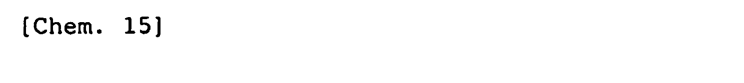

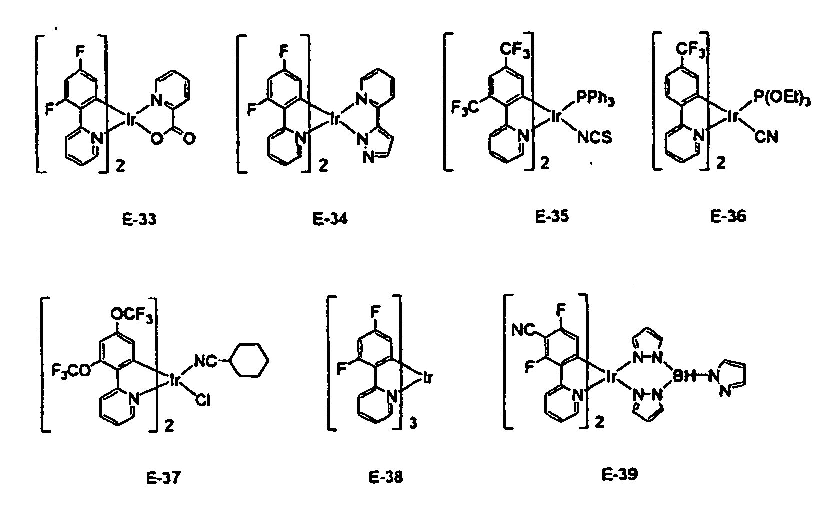

In this case, the light-emitting layer preferably contains the phosphorescent low-molecular weight compound at 1 to 50 parts by weight, and more preferably 5 to 20 parts by weight based on 100 parts by weight of the polymer compound (III). When the light-emitting layer further contains the electron transport compound, the content of the electron transport compound is preferably 5 to 95 parts by weight, and more preferably 20 to 80 parts by weight based on 100 parts by weight of the polymer compound (III).

Similar to the polymer compound (III), the polymer compound (IV) obtained by polymerizing the compounds (A) and (C) are used in combination with a phosphorescent low-molecular weight compound to form a light-emitting layer. In this case, the light-emitting layer preferably contains the phosphorescent low-molecular weight compound at 1 to 50 parts by weight, and more preferably 5 to 20 parts by weight based on 100 parts by weight of the polymer compound (IV).

When the light-emitting layer is produced from the polymer compound (I), the process for producing the light-emitting layer is not particularly limited and may be performed as follows. The polymer compound (I) and optionally an electron transport compound as required are dissolved in a solvent to give a solution. The solvents used herein are not particularly limited and examples thereof include chlorine solvents such as chloroform, methylene chloride and dichloroethane, ether solvents such as tetrahydrofuran and anisole, aromatic hydrocarbon solvents such as toluene and xylene, ketone solvents such as acetone and methyl ethyl ketone, and ester solvents such as ethyl acetate, butyl acetate and ethyl cellosolve acetate. Subsequently, the solution is spread on a substrate by wet film-forming methods such as spin coating, casting, microgravure coating, gravure coating, bar coating, roll coating, wire bar coating, dip coating, spray coating, screen printing, flexographic printing, offset printing and inkjet printing. In the case of spin coating or dip coating, the solution preferably contains the solvent at 1000 to 20000 parts by weight based on 100 parts by weight of the polymer compound (I), but the solvent amount is variable depending on the compound used or film-making conditions. When the electron transport compound is used, the solution preferably contains the compound at 10 to 900 parts by weight based on 100 parts by weight of the polymer compound (I).

The light-emitting layer may be formed from the polymer compound (II) in a manner similar to the polymer compound (I). For example, in the case of spin coating or dip coating, the solution preferably contains the solvent at 1000 to 20000 parts by weight based on 100 parts by weight of the polymer compound (II).

The light-emitting layer may be produced from the polymer compound (III) as follows. The polymer compound (III), a phosphorescent low-molecular weight compound and optionally an electron transport compound as required are dissolved in a solvent to give a solution. The solvents used herein are as described hereinabove. The solution is spread on a substrate by methods as described above. In the case of spin coating or dip coating, the solution preferably contains the phosphorescent low-molecular weight compound at 1 to 50 parts by weight, and the solvent at 1000 to 20000 parts by weight based on 100 parts by weight of the polymer compound (III), but these amounts are variable depending on the compounds used or film-making conditions. When the electron transport compound is used, the solution preferably contains the compound at 10 to 900 parts by weight based on 100 parts by weight of the polymer compound (III).

The light-emitting layer may be formed from the polymer compound (IV) in a manner similar to the polymer compound (III) . For example, in the case of spin coating or dip coating, the solution preferably contains the phosphorescent low-molecular weight compound at 1 to 50 parts by weight, and the solvent at 1000 to 20000 parts by weight based on 100 parts by weight of the polymer compound (IV).

### 2. Organic light-emitting devices

The organic light-emitting devices of the present invention have at least one organic layer sandwiched between anode and cathode, and at least one of the at least one organic layer is the specific light-emitting layer as described hereinabove. According to the present invention, the light-emitting layer may be formed by simple methods as mentioned above, and the organic light-emitting devices may be produced with an increased area.

Fig. 1 shows an embodiment of the organic light-emitting devices according to the present invention, but the constitution of the organic light-emitting devices of the present invention is not limited thereto. In Fig. 1, a hole transport layer (3), a light-emitting layer (4) as described above and an electron transport layer (5) are provided in this order between an anode (2) on a transparent substrate (1) and a cathode (6). In the organic light-emitting devices, the layers provided between the anode (2) and the cathode (6) may be: 1) the hole transport layer/the light-emitting layer; 2) the light-emitting layer/the electron transport layer; or 3) the light-emitting layer alone. Further, two or more light-emitting layers as described above may be provided.

When the light-emitting layer has hole transport properties, electron transport properties and phosphorescence emitting properties, the organic light-emitting device shows high luminous efficiency and durability without other organic layers. Further, such light-emitting layers allow for simplification of production process.

Each of the layers may contain a high-molecular material mixed therein as a binder. Examples of the high-molecular materials include polymethyl methacrylate, polycarbonates, polyesters, polysulfones and polyphenylene oxides.

The hole transport layer and the electron transport layer may be formed from a hole transport compound and an electron transport compound alone, respectively, or may be each formed from a mixture of such materials differing in functions. To increase carrier transport properties, the light-emitting layer may contain electron transport compounds as described hereinabove and other hole transport compounds in addition to the polymer compound contained in the device according to the present invention. Such compounds may be low-molecular weight compounds or polymer compounds.

Examples of the hole transport compounds for forming the hole transport layer and the hole transport compounds to be mixed in the light-emitting layer include TPD (N,N'-dimethyl-N,N'-(3-methylphenyl)-1,1'-biphenyl-4,4'-diamine); α-NPD (4,4'-bis[N-(1-naphthyl)-N-phenylamino]biphenyl); low-molecular weight triphenylamine derivatives such as m-MTDATA (4,4',4" -tris(3-methylphenylphenylamino)triphenylamine); polyvinylcarbazole; polymer compounds obtained by polymerizing a monomer in which a polymerizable functional group is introduced into the triphenylamine derivative; and fluorescent polymer compounds such as polyparaphenylenevinylene and polydialkylfluorene. Examples of the above polymer compounds include polymer compounds having a triphenylamine skeleton as disclosed in JP-A-H08-157575. The hole transport compounds may be used singly, or two or more kinds may be used in combination. Further, differing hole transport compounds may be laminated. The thickness of the hole transport layer depends on conductivity but is preferably 1 nm to 5 µm, more preferably 5 nm to 1 µm, and particularly preferably 10 nm to 500 nm.

Examples of the electron transport compounds for forming the electron transport layer and the electron transport compounds to be mixed in the light-emitting layer include low-molecular weight compounds such as quinolinol derivative metal complexes such as Alq₃ (aluminum trisquinolinolate), oxadiazole derivatives, triazole derivatives, imidazole derivatives, triazine derivatives and triarylborane derivatives; and polymer compounds obtained by polymerizing a monomer in which a polymerizable functional group introduced into the low molecular weight compound. Examples of the above polymer compounds include poly-PBD disclosed in JP-A-H10-1665. The electron transport compounds may be used singly, or two or more kinds may be used in combination. Further, differing electron transport compounds may be laminated. The thickness of the electron transport layer depends on conductivity but is preferably 1 nm to 5 µm, more preferably 5 nm to 1 µm, and particularly preferably 10 nm to 500 nm.

A hole blocking layer may be provided adjacent to the light-emitting layer on the cathode side to block the passage of holes through the light-emitting layer and to permit the holes and electrons to recombine efficiently in the light-emitting layer. The hole blocking layer may be formed from known materials such as triazole derivatives, oxadiazole derivatives and phenanthroline derivatives.

In order to lower the hole-injection barrier, a buffer layer may be provided between the anode and the hole transport layer or between the anode and an organic layer placed adjacent to the anode. The buffer layer may be formed from known materials such as copper phthalocyanine and polyethylene dioxythiophene/polystyrenesulfonic acid mixture (PEDOT/PSS).

To enhance electron injection efficiency, an insulating layer 0.1 to 10 nm in thickness may be provided between the cathode and the electron transport layer or between the cathode and an organic layer placed adjacent to the cathode. The insulating layer may be formed from known materials such as lithium fluoride, sodium fluoride, magnesium fluoride, magnesium oxide and alumina.

In the organic light-emitting devices according to the present invention, known transparent conductive materials may be suitably used as anode materials, with examples including ITO (indium tin oxide), tin oxide, zinc oxide and conductive polymers such as polythiophene, polypyrrole and polyaniline. The electrodes formed from these transparent conductive materials preferably have a surface resistance of 1 to 50 Ω/□ (ohm/square). The thickness of the anode is preferably 50 to 300 nm.

In the organic light-emitting devices of the invention, known cathode materials may be suitably used, with examples including alkali metals such as Li, Na, K and Cs; alkaline earth metals such as Mg, Ca and Ba; Al; MgAg alloy; and alloys of Al and alkali metals or alkaline earth metals such as AlLi and AlCa. The thickness of the cathode is preferably 10 nm to 1 µm, and more preferably 50 to 500 nm. When highly active metals such as alkali metals or alkaline earth metals are used, the cathode thickness is preferably 0.1 to 100 nm, and more preferably 0.5 to 50 nm; in this case, a metal layer that is stable in the atmosphere is provided on the cathode to protect the cathode metal. Examples of the metals for forming the metal layer include Al, Ag, Au, Pt, Cu, Ni and Cr. The thickness of the metal layer is preferably 10 nm to 1 µm, and more preferably 50 to 500 nm.

The substrate in the organic light-emitting devices of the invention may be suitably an insulating substrate that is transparent at an emission wavelength of the light-emitting material. Specific examples include glass and transparent plastics such as PET (polyethylene terephthalate) and polycarbonate.

The hole transport layer and the electron transport layer may be formed by dry film-forming methods such as resistance thermal deposition, electron beam deposition and sputtering; and wet film-forming methods such as spin coating, casting, microgravure coating, gravure coating, bar coating, roll coating, wire bar coating, dip coating, spray coating, screen printing, flexographic printing, offset printing and inkjet printing. The dry film-forming methods are suitably used for low-molecular weight compounds, and the wet film-forming methods are suited for polymer compounds.

The anode materials may be formed into films by electron beam deposition, sputtering, chemical reactions and coating methods. The cathode materials may be formed into films by resistance thermal deposition, electron beam deposition, sputtering and ion plating methods.

### 3. Uses

The organic light-emitting devices of the present invention may be used as pixels in matrix or segment displays according to known methods. The organic light-emitting devices may be suitably used as surface emitting light sources without forming pixels.

Specifically, the organic light-emitting devices may be suitably used in displays, backlights, electrophotography, illuminating light sources, recording light sources, exposure light sources, reading light sources, indicators, signs, interiors and optical communication.

### [EXAMPLES]

The present invention will be described in detail herein below.

Polymer compounds were analyzed by the following methods.

### (1) Molecular weight

The molecular weight was determined with a gel permeation chromatograph (GPC) under the following conditions.
Columns: Shodex KF-G + KF804L + KF802 + KF801
Eluting solution: tetrahydrofuran (THF)
Temperature: 40°C
Detector: RI (Shodex RI-71)

### (2) Chemical composition

¹³C -NMR was performed under the following conditions.
Apparatus: JNM EX270 manufactured by JEOL Ltd.
67.5 MHz
Solvent: deuterated chloroform
ICP elemental analysis was carried out under the following conditions.
Apparatus: ICPS 8000 manufactured by Shimadzu Corporation (3) Glass transition temperature (Tg)
Approximately 7 mg of the sample was weighed out and was tested on a differential scanning calorimeter (SSC-5200 manufactured by Seiko Instruments Inc.). An aluminum pan was used as a reference, and the temperature increase rate was 10°C/min.

The external quantum efficiency, maximum brightness and brightness half-life of the devices obtained were measured by the following methods.

### (4) External quantum efficiency

The organic light-emitting device was placed in the dark. A spectroradiometer (CS-1000T manufactured by Konica Minolta Holdings, Inc.) was located 100 cm away from the emitting surface in a perpendicular direction. A predetermined voltage was applied to the organic light-emitting device for 1 second to cause the device to emit light. The current passing through the device, the front brightness observed on the anode side of the device, and an emission spectrum were measured at a viewing angle of 0.2 degree. The voltage was increased stepwise by 0.1 V starting from 0 V. The current, brightness and emission spectrum were measured immediately after the voltage was increased. The external quantum efficiency was calculated from the data obtained, and the maximum value was obtained as the external quantum efficiency of the device.

### (5) Maximum brightness

The front brightness was measured in the same manner as the measurement of the external quantum efficiency except that the voltage was increased stepwise by 0.5 V. The maximum value was obtained as the maximum brightness of the device.

### (6) Brightness half-life

The organic light-emitting device was energized to a brightness of 100 cd/m² while the front brightness was measured in the same manner as the measurement of the external quantum efficiency. A silicon photodiode was attached to the device on the anode side. While the current was maintained constant, the photocurrent through the photodiode was measured. The time required until the photocurrent was reduced by half was determined as the brightness half-life.

### [Synthetic Example 1]

### Synthesis of compound (A1)

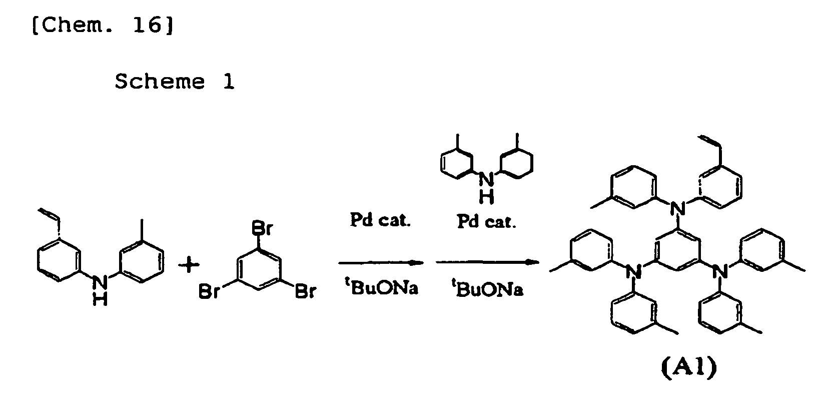

A mixture consisting of 1.06 g (5.0 mmol) of 3-tolyl-3-styrylamine, 1.65 g (5.3 mmol) of 1,3,5-tribromobenzene, 22.5 mg (0.1 mmol) of palladium (II) acetate, 143 mg (0.3 mmol) of 2-dicyclohexylphosphino-2',4',6'-triisopropylbiphenyl, 1.06g (11 mmol) of t-butoxysodium and 30 ml of toluene was heated under reflux for 2 hours. To the reaction solution, 1.89 g (10.1 mmol) of ditolylamine, 2.114 g (22 mmol) of t-butoxysodium and 15 ml of toluene were added, and the mixture was heated under reflux for another 2 hours. The resultant reaction solution was cooled to room temperature and was extracted with ethyl acetate. The extract was purified by silica gel chromatography to afford 2.5 g (3.7 mmol) of a vinyl monomer.

The compound (A1) was identified and the following data was obtained.
Elemental analysis: Theoretical values (C₄₉H₄₅N₃) : C: 87.07, H: 6.71, N: 6.22. Measured values: C: 87.15, H: 6.81, N: 6.04.
Mass spectroscopy (EI): 676 (M⁺)

### [Synthetic Example 2]

### Synthesis of compound (A2)

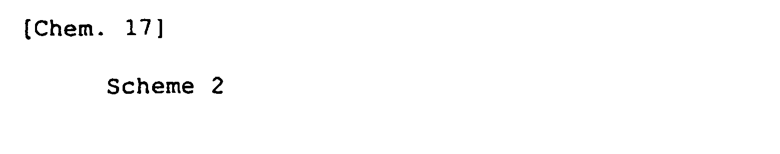

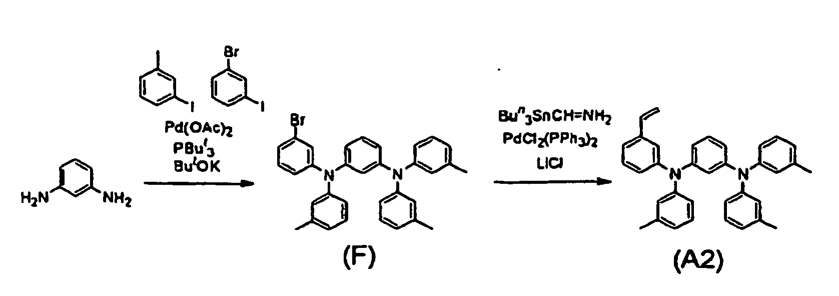

A mixture consisting of 20 g (0.18 mol) of m-phenylenediamine, 81 g (0.37 mol) of 3-iodotoluene, 0.50 g (2.2 mmol) of palladium acetate, 1.4 g (6.9 mmol) of tri-t-butylphosphine, 45 g (0.40 mol) of potassium-t-butoxide and 300 ml of xylene was heated under reflux for 3 hours. The resultant reaction solution was cooled to room temperature. Further, 40 g (0.18 mol) of 3-iodotoluene and 52 g (0.18 mol) of 3-bromoiodobenzene were added, and the mixture was heated under reflux for another 3 hours. The resultant reaction liquid was filtered and the solvent was distilled off under reduced pressure. The distillate was purified by silica gel column chromatography to afford 2.7 g (5.1 mmol) of a compound (F).

A mixture consisting of 2.0 g (3.7 mmol) of the compound (F), 1.3 g (4.1 mmol) of tri-n-butyl(vinyl)tin, 0.10 g (0.14 mmol) of dichlorobis(triphenylphosphine)palladium, 0.17 g (4.0 mmol) of lithium chloride and 30 ml of toluene was heated under reflux for 4 hours. The resultant reaction mixture was filtered and thereby insolubles were removed. The filtrate was distilled under reduced pressure to remove the solvent. The distillate was purified by silica gel column chromatography to afford 1.5 g (3.1 mmol) of a compound (A2).

The compound (A2) was identified and the following data was obtained.
Elemental analysis: Theoretical values (C₃₅H₃₂N₂) : C: 87.46, H: 6.71, N: 5.83. Measured values: C: 87.59, H: 6.91, N: 5.50.
Mass spectroscopy (EI): 480 (M⁺)

### [Example 1]

### Synthesis of polymer compound (1-1)

An airtight container was charged with 200 mg of the compound (A1) and 2.4 ml of dehydrated toluene. Subsequently, a toluene solution (0.1 M, 47 µl) of V-601 (manufactured by Wako Pure Chemical Industries Ltd.) was added, followed by five cycles of freezing and degassing. The evacuated container was tightly closed. The materials were stirred at 60°C for 60 hours. After the reaction, the reaction liquid was added dropwise to 100 ml of acetone, and a precipitate resulted. The product was purified by being reprecipitated twice from toluene into acetone, and was dried at 50°C in vacuo overnight to afford a polymer compound (1-1). The polymer compound (1-1) had a weight average molecular weight (Mw) of 30500 and a molecular weight distribution index (Mw/Mn) of 1.71. The glass transition temperature Tg of the polymer compound (1-1) was 180.2°C.

### [Example 2]

### Synthesis of polymer compound (2-1)

An airtight container was charged with 100 mg of the compound (A1), 100 mg of a triarylborane derivative (compound (G)) and 2.4 ml of dehydrated toluene. Subsequently, a toluene solution (0.1 M, 47 µl) of V-601 (manufactured by Wako Pure Chemical Industries Ltd.) was added, followed by five cycles of freezing and degassing. The evacuated container was tightly closed. The materials were stirred at 60°C for 60 hours. After the reaction, the reaction liquid was added dropwise to 100 ml of acetone, and a precipitate resulted. The product was purified by being reprecipitated twice from toluene into acetone, and was dried at 50°C in vacuo overnight to afford a polymer compound (2-1). The polymer compound (2-1) had a weight average molecular weight (Mw) of 64500 and a molecular weight distribution index (Mw/Mn) of 2.01. From the ICP elemental analysis and ¹³C-NMR analysis, the ratios x/n and y/n in the polymer compound were estimated to be 0.46 and 0.54, respectively. The glass transition temperature Tg of the polymer compound (2-1) was 183.2°C.

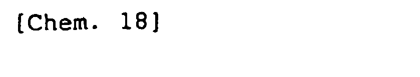

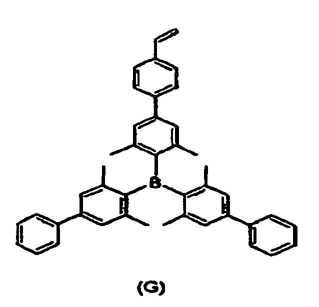

### [Reference Example 1]

### Synthesis of polymer compound (1-2)

A polymer compound (1-2) was produced in the same manner as in Example 1 except that the compound (A1) was replaced by the compound (A2). The polymer compound (1-2) had a weight average molecular weight (Mw) of 41700 and a molecular weight distribution index (Mw/Mn) of 1.74. The glass transition temperature Tg of the polymer compound (1-2) was 159.7°C.

### [Reference Example 2]

### Synthesis of polymer compound (2-2)

A polymer compound (2-2) was produced in the same manner as in Example 2 except that the compound (A1) was replaced by the compound (A2). The polymer compound (2-2) had a weight average molecular weight (Mw) of 92000 and a molecular weight distribution index (Mw/Mn) of 2.40. From the ICP elemental analysis and ¹³C-NMR analysis, the ratios x/n and y/n in the polymer compound were estimated to be 0.44 and 0.56, respectively. The glass transition temperature Tg of the polymer compound (2-2) was 170.0°C.

### [Example 3]

### Fabrication and evaluation of organic light-emitting device

An ITO glass substrate (NIPPO ELECTRIC CO., LTD.) was used. The glass substrate was a 25 mm square, and two stripe electrodes (anodes) of ITO (indium tin oxide) were formed with a width of 4 mm on one surface of the substrate.

The ITO glass substrate was spin coated with poly(3,4-ethylenedioxythiophene) polystyrenesulfonic acid (product name: Baytron P manufactured by Bayer AG) at 3500 rpm for 40 seconds. The coated substrate was dried in a vacuum dryer at reduced pressure and 60°C for 2 hours, whereby an anode buffer layer having a thickness of about 50 nm was formed. Subsequently, 40.5 mg of the polymer compound (1-1), 9 mg of a compound (H) and 40.5 mg of a compound (J) were dissolved in 2910 mg of toluene (special grade, manufactured by Wako Pure Chemical Industries, Ltd.). The solution was filtered through a 0.2 µm filter and the filtrate was obtained as a coating solution. The coating solution was spread over the anode buffer layer by spin coating at 3000 rpm for 30 seconds. The coating was dried at room temperature (25°C) for 30 minutes to form a light-emitting layer, which was approximately 100 nm in thickness.

The substrate with the light-emitting layer was placed in a deposition apparatus. Barium and aluminum were codeposited in a weight ratio of 1:10 such that two stripe cathodes were formed with a width of 3 mm and in a direction perpendicular to the anode-extending direction. The cathodes were approximately 50 nm thick.

Thereafter, leads (wires) were attached to the anodes and cathodes in an argon atmosphere. As a result, four organic EL devices 4 mm in length and 3 mm in width were manufactured. The organic EL devices were energized using a programmable direct voltage/current source (TR6143 manufactured by ADVANTEST CORPORATION) to emit light.

Table 1 sets forth the maximum external quantum efficiency, maximum attainable brightness and brightness half-life from the initial brightness 100 cd/m² at a constant current.

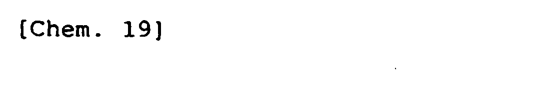

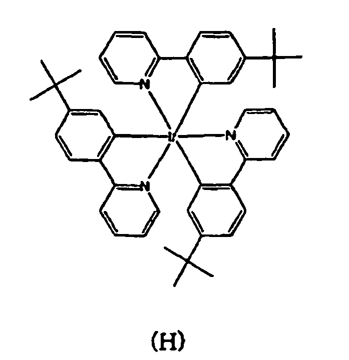

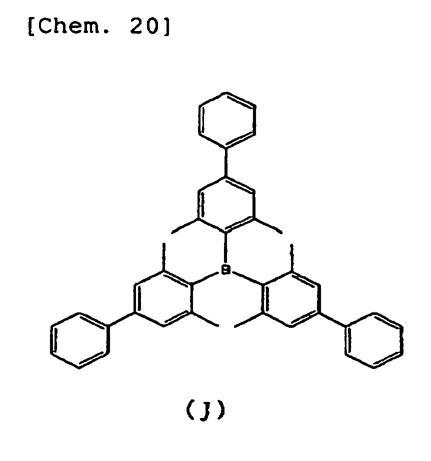

### [Example 4]

### Fabrication and evaluation of organic light-emitting device

Organic light-emitting devices were manufactured in the same manner as in Example 3 except that the coating solution for forming the light-emitting layer was changed, in detail, 40. 5 mg of the polymer compound (1-1) , 9 mg of the compound (H) , 40.5 mg of the compound (J) and 2910 mg of toluene were replaced by 81. 0 mg of the polymer compound (2-1), 9.0 mg of the compound (H) and 2910 mg of toluene. Table 1 sets forth the maximum external quantum efficiency, maximum brightness and brightness half-life from the initial brightness 100 cd/m² at a constant current.

### [Reference Example 3]

### Fabrication and evaluation of organic light-emitting device

Organic light-emitting devices were manufactured in the same manner as in Example 3 except that the coating solution for forming the light-emitting layer was changed, in detail, 40.5 mg of the polymer compound (1-1), 9 mg of the compound (H), 40.5 mg of the compound (J) and 2910 mg of toluene were replaced by 40.5 mg of the polymer compound (1-2), 9.0 mg of the compound (H), 40.5 mg of the compound (J) and 2910 mg of toluene. Table 1 sets forth the maximum external quantum efficiency, maximum brightness and brightness half-life from the initial brightness 100 cd/m² at a constant current.

### [Reference Example 4]

### Fabrication and evaluation of organic light-emitting device

Organic light-emitting devices were manufactured in the same manner as in Example 3 except that the coating solution for forming the light-emitting layer was changed, in detail, 40.5 mg of the polymer compound (1-1) , 9 mg of the compound (H) , 40.5 mg of the compound (J) and 2910 mg of toluene were replaced by 81.0 mg of the polymer compound (2-2), 9.0 mg of the compound (H) and 2910 mg of toluene. Table 1 sets forth the maximum external quantum efficiency, maximum brightness and brightness half-life from the initial brightness 100 cd/m² at a constant current.

**[Table 1]**

| | Polymer compound | Low-molecular weight compound | Maximum external quantum efficiency (%) | Maximum attainable brightness (cd/m²) | Brightness half-life (h) |
|---|---|---|---|---|---|
| Ex. 3 | 1-1 | H, J | 8.2 | 45000 | 5100 |
| Ex. 4 | 2-1 | H | 8.1 | 50000 | 5400 |
| Ref. Ex. 3 | 1-2 | H, J | 5.6 | 39000 | 2600 |
| Ref. Ex. 4 | 2-2 | H | 5.7 | 44000 | 2100 |

## Claims

1. An organic light-emitting device that includes at least one organic layer sandwiched between anode and cathode wherein at least one of the at least one organic layer is a light-emitting layer that comprises a polymer compound including structural units from a polymerizable compound (A) of Formula (1) below: 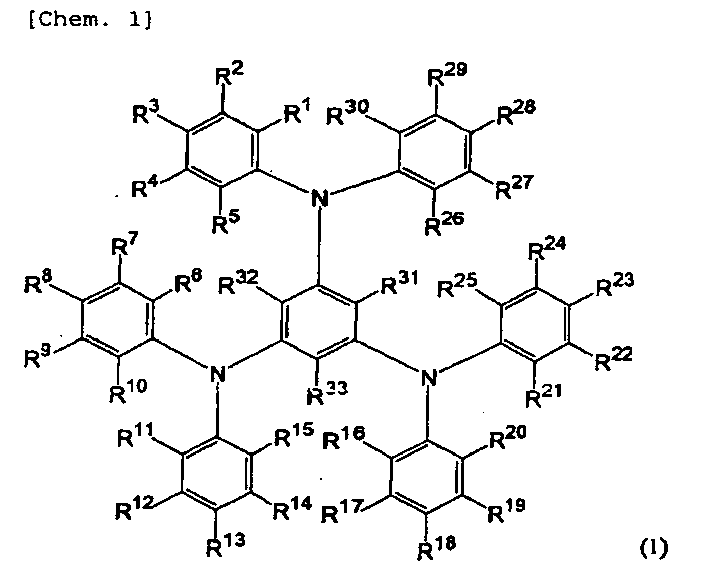 wherein R²⁹ is a substituent group having an alkenyl group group, R⁴, R⁹, R¹⁴, R¹⁹ and R²² are an atom or a substituent group selected from the group consisting of halogen atoms, a cyano group, C1-10 alkyl groups, C6-10 aryl groups, amino groups optionally substituted with a C1-10 alkyl group, C1-10 alkoxy groups and silyl groups, and the substituent groups other than R²⁹, R⁴, R⁹, R¹⁴, R¹⁹, abd R²², are hydrogen atom.

2. The organic light-emitting device according to claim 1, wherein the polymer compound further includes structural units from a phosphorescent polymerizable compound (B) .

3. The organic light-emitting device according to claim 1, wherein the polymer compound further includes structural units from a phosphorescent polymerizable compound (B) and structural units from an electron transport polymerizable compound (C).

4. The organic light-emitting device according to claim 2 or 3, wherein the phosphorescent polymerizable compound (B) is a complex represented by Formula (2-1) below: 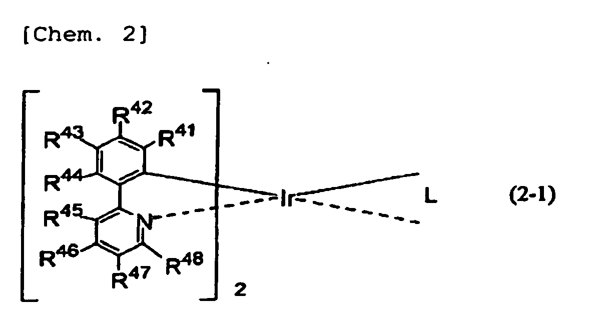 wherein R⁴¹ to R⁴⁸ are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; a pair of R⁴¹ and R⁴² , a pair of R⁴² and R⁴³ , a pair of R⁴³ and R⁴⁴, a pair of R⁴⁴ and R⁴⁵, a pair of R⁴⁵ and R⁴⁶, pair of R⁴⁶ and R⁴⁷, or a pair of R⁴⁷ and R⁴⁸ may be linked together to form a condensed ring; and L is a bidentate ligand selected from Formulae (2-2) to (2-4) below: 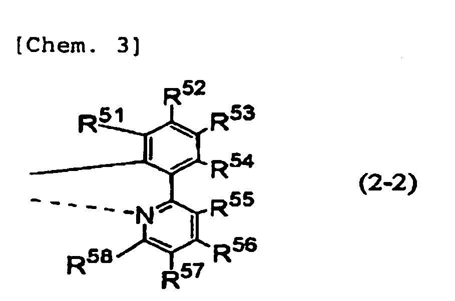 wherein at least one of R⁵¹ to R⁵⁸ is a substituent group having a polymerizable functional group, and those of R⁵¹ to R⁵⁸ that are not substituent groups having a polymerizable functional group are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; and a pair of R⁵¹ and R⁵², a pair of R⁵² and R⁵³, a pair of R⁵³ and R⁵⁴, pair of R⁵⁴ and R⁵⁵, **a** pair of R⁵⁵ and R⁵⁶, a pair of R⁵⁶ and R⁵⁷, or a pair of R⁵⁷ and R⁵⁸ may be linked together to form a condensed ring; 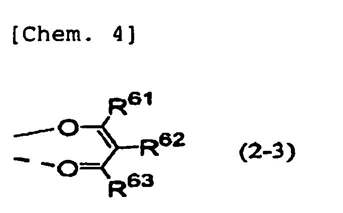 wherein at least one of R⁶¹ to R⁶³ is a substituent group having a polymerizable functional group, and those of R⁶¹ to R⁶³ that are not substituent groups having a polymerizable functional group are each independently an atom or a substituent group selected from the group consisting of a hydrogen atom, a cyano group, C1-10 alkyl groups, C6-10 aryl groups, amino groups optionally substituted with a C1-10 alkyl group, C1-10 alkoxy groups and silyl groups; and a pair of R⁶¹ and R⁶² or a pair of R⁶² and R⁶³ may be linked together to form a condensed ring; 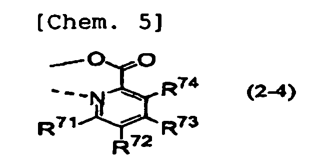 wherein at least one of R⁷¹ to R⁷⁴ is a substituent group having a polymerizable functional group, and those of R⁷¹ to R⁷⁴ that are not substituent groups having a polymerizable functional group are each independently an atom or a substituent group selected from the group consisting of a hydrogen atom, halogen atoms, a cyano group, C1-10 alkyl groups, C6-10 aryl groups, amino groups optionally substituted with a C1-10 alkyl group, C1-10 alkoxy groups and silyl groups; and a pair of R⁷¹ and R⁷², a pair of R⁷² and R⁷³, or a pair of R⁷³ and R⁷⁴ may be linked together to form a condensed ring.

5. The organic light-emitting device according to claim 1, wherein the light-emitting layer further comprises a phosphorescent compound.

6. The organic light-emitting device according to claim 1, wherein the light-emitting layer further comprises a phosphorescent compound and the polymer compound further includes structural units from an electron transport polymerizable compound (C).

7. The organic light-emitting device according to claim 3 or 6, wherein the electron transport polymerizable compound (C) is an oxadiazole derivative or a triarylborane derivative.

8. A surface-emitting light source wherein the organic light-emitting device described in any one of claims 1 7 is light-emitting device described in any one of claims 1 to 7 is used.

9. An image display wherein the organic light-emitting device described in any one of claims 1 to 7 is used.

## Patentansprüche

1. Organische lichtemittierende Vorrichtung, die mindestens eine organische Schicht aufweist, die zwischen Anode und Kathode eingefasst ist, wobei mindestens eine der mindestens einen organischen Schicht eine lichtemittierende Schicht ist, die eine Polymerverbindung enthält, die strukturelle Einheiten aus einer polymerisierbaren Verbindung (A) der nachstehenden Formel (1) umfasst: 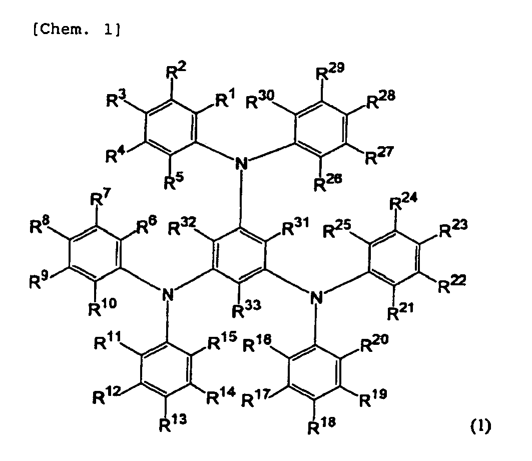 worin R²⁹ ein Substituent mit einer Alkenylgruppe ist, R⁴, R⁹, R¹⁴, R¹⁹ und R²² ein Atom oder eine Substituentengruppe ist, die aus der Gruppe ausgewählt ist, die aus Halogenatomen, einer Cyangruppe, C1-C10-Alkylgruppen C6-C10-Arylgruppen, gegebenenfalls mit einer C1-C10-Alkylgruppe substituierten Amingruppen, C1-C10-Alkoxygruppen und Silylgruppen besteht, und die anderen Substituentengruppen als R²⁹, R⁴, R⁹, R¹⁴, R¹⁹ und R²² Wasserstoffatome sind.

2. Organische lichtemittierende Vorrichtung nach Anspruch 1, wobei die Polymerverbindung außerdem strukturelle Einheiten aus einer phosphoreszierenden polymerisierbaren Verbindung (B) aufweist.

3. Organische lichtemittierende Vorrichtung nach Anspruch 1, wobei die Polymerverbindung außerdem strukturelle Einheiten aus einer phosphoreszierenden polymerisierbaren Verbindung (B) und strukturelle Einheiten aus einer polymerisierbaren Elektronentransportverbindung (C) aufweist.

4. Organische lichtemittierende Vorrichtung nach Anspruch 2 oder 3, wobei die phosphoreszierende polymerisierbare Verbindung (B) ein Komplex der folgenden Formel (2-1) ist: 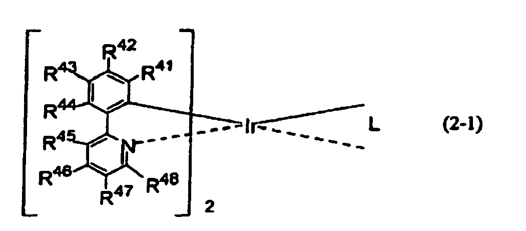 worin R⁴¹ bis R⁴⁸ jeweils unabhängig voneinander ein Atom oder eine Substituentengruppe darstellen, die aus der Gruppe ausgewählt ist, die aus einem Wasserstoffatom, Halogenatomen, einer Cyangruppe, C1-C10-Alkylgruppen, C6-C10-Arylgruppen, gegebenenfalls mit einer C1-C10-Alkylgruppe substituierten Amingruppen, C1-C10-Alkoxygruppe und Silylgruppen besteht; ein Paar aus R⁴¹ und R⁴², und ein Paar aus R⁴² und R⁴³, ein Paar aus R⁴³ und R⁴⁴, und ein Paar aus R⁴⁴ und R⁴⁵, ein Paar aus R⁴⁵ und R⁴⁶, ein Paar aus R⁴⁶ und R⁴⁷ oder ein Paar aus R⁴⁷ und R⁴⁸ unter Bildung eines kondensierten Rings miteinander verbunden sein kann; und L ein zweizähniger Ligand ist, der unter den folgenden Formeln (2-2) bis (2-4) ausgewählt ist: 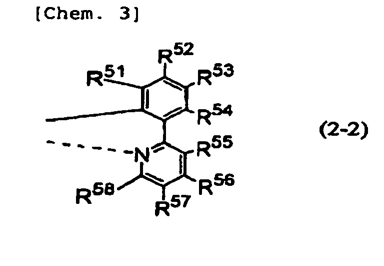 worin mindestens einer der Substituenten R⁵¹ bis R⁵⁸ eine Substituentengruppe mit einer polymerisierbaren funktionellen Gruppe ist, und solche Substituenten von R⁵¹ bis R⁵⁸, die nicht Substituentengruppen mit einer polymerisierbaren funktionellen Gruppe sind, unabhängig voneinander ein Atom oder eine Substituentengruppe sind, die aus der Gruppe ausgewählt ist, die aus einem Wasserstoffatom, Halogenatomen, einer Cyangruppe, C1-C10-Alkylgruppen, C6-C10-Arylgruppen, gegebenenfalls mit einer C1-C10-Alkylgruppe substituierten Amingruppen, C1-C10-Alkoxygruppen und Silylgruppen besteht; und ein Paar aus R⁵¹ und R⁵², ein Paar aus R⁵² und R⁵³, ein Paar aus R⁵³ und R⁵⁴, ein Paar aus R⁵⁴ und R⁵⁵, ein Paar aus R⁵⁵ und R⁵⁶, ein Paar aus R⁵⁶ und R⁵⁷, oder ein Paar aus R⁵⁷ und R⁵⁸ unter Bildung eines kondensierten Rings miteinander verbunden sein kann; 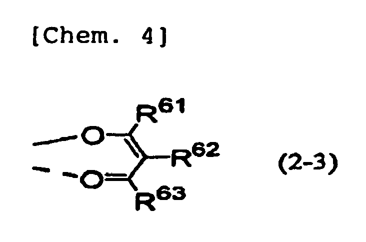 worin mindestens einer der Substituenten R⁶¹ bis R⁶³ eine Substituentengruppe mit einer polymerisierbaren funktionellen Gruppe ist, und solche Substituenten von R⁶¹ bis R⁶³, die nicht Substituentengruppen mit einer polymerisierbaren funktionellen Gruppe sind, jeweils unabhängig voneinander ein Atom oder eine Substituentengruppe sind, die aus der Gruppe ausgewählt ist, die aus einem Wasserstoffatom, einer Cyangruppe, C1-C10-Alkylgruppen, C6-C10-Arylgruppen, gegebenenfalls mit einer C1-C10-Alkylgruppe substituierten Amingruppen, C1-C10-Alkoxygruppen und Silylgruppen besteht; und ein Paar aus R⁶¹ und R⁶² oder ein Paar aus R⁶² und R⁶³ unter Bildung eines kondensierten Rings miteinander verbunden sein kann; 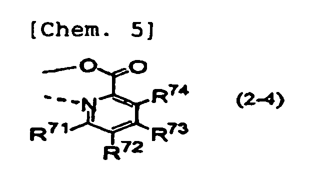 worin mindestens einer der Substituenten R⁷¹ bis R⁷⁴ eine Substituentengruppe mit einer polymerisierbaren funktionellen Gruppe ist, und solche Substituenten aus R⁷¹ bis R⁷⁴, die nicht Substituentengruppen mit einer polymerisierbaren funktionellen Gruppe sind, unabhängig voneinander ein Atom oder eine Substituentengruppe sind, die aus der Gruppe ausgewählt ist, die aus einem Wasserstoffatom, einem Halogenatom, einer Cyangruppe, C1-C10-Alkylgruppen, C6-C10-Arylgruppen, gegebenenfalls mit einer C1-C10-Alkylgruppe substituierten Amingruppen, C1-C10-Alkoxygruppe und Silylgruppen besteht; und ein Paar aus R⁷¹ und R⁷², ein Paar aus R⁷² und R⁷³ oder ein Paar aus R⁷³ und R⁷⁴ unter Bildung eines kondensierten Rings miteinander verbunden sein kann.

5. Organische lichtemittierende Vorrichtung nach Anspruch 1, wobei die lichtemittierende Schicht außerdem eine phosphoreszierende Verbindung enthält.

6. Organische lichtemittierende Vorrichtung nach Anspruch 1, wobei die lichtemittierende Schicht außerdem eine phosphoreszierende Verbindung enthält und die Polymerverbindung außerdem strukturelle Einheiten aus einer polymerisierbaren Elektronentransportverbindung (C) aufweist.

7. Organische lichtemittierende Vorrichtung nach Anspruch 3 oder 6, wobei die polymerisierbare Elektronentransportverbindung (C) ein Oxadiazolderivat oder ein Triarylboranderivat ist.

8. Oberflächenemittierende Lichtquelle, wobei die organische lichtemittierende Vorrichtung nach einem der Ansprüche 1 bis 7 verwendet wird.

9. Bildanzeigevorrichtung, wobei die organische lichtemittierende Vorrichtung nach einem der Ansprüche 1 bis 7 verwendet wird.

## Revendications

1. Dispositif organique émettant de la lumière qui comprend au moins une couche organique prise en sandwich entre une anode et une cathode, dans lequel au moins une de la au moins une couche organique est une couche émettant de la lumière qui comprend un composé polymère comprenant des unités structurelles d'un composé polymérisable (A) de la formule (1) ci-dessous : 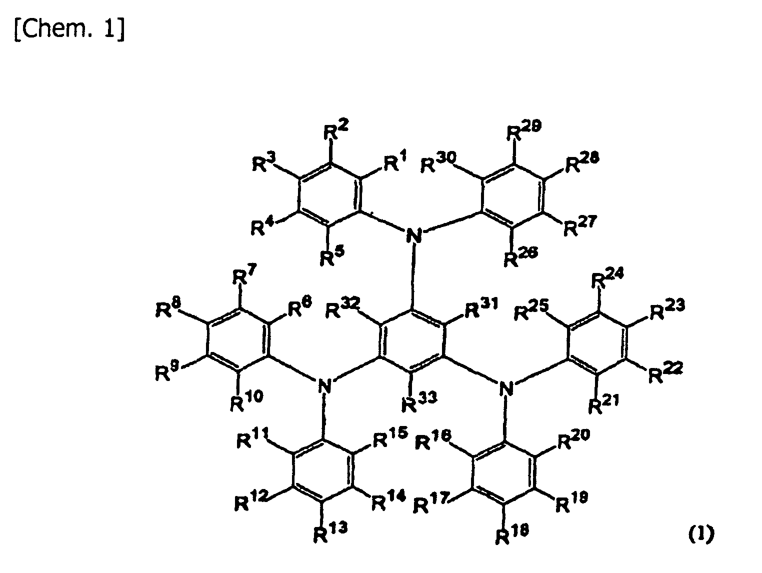 dans laquelle R²⁹ est un groupe substituant présentant un groupe alcényle, R⁴, R⁹, R¹⁴, R¹⁹ et R²² sont un atome ou un groupe substituant choisi dans le groupe constitué d'atomes d'halogène, d'un groupe cyano, de groupes alkyle en C1-10, de groupes aryle en C6-10, de groupes amino éventuellement substitués avec un groupe alkyle en C1-10, de groupes alcoxy en C1-10 et de groupes silyle, et les groupes substituants différents de R²⁹, R⁴, R⁹, R¹⁴, R¹⁹ et R²² sont un atome d'hydrogène.

2. Dispositif organique émettant de la lumière selon la revendication 1, dans lequel le composé polymère comprend de plus des unités structurelles d'un composé polymérisable phosphorescent (B).

3. Dispositif organique émettant de la lumière selon la revendication 1, dans lequel le composé polymère comprend de plus des unités structurelles d'un composé polymérisable phosphorescent (B) et des unités structurelles d'un composé polymérisable de transport d'électrons (C).

4. Dispositif organique émettant de la lumière selon la revendication 2 ou 3, dans lequel le composé polymérisable phosphorescent (B) est un complexe représenté par la formule (2-1) ci-dessous : 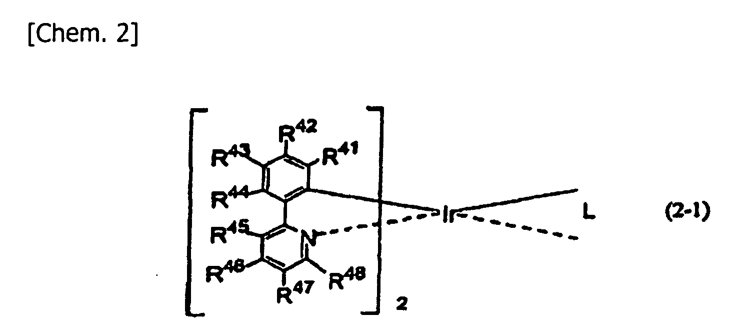 dans laquelle R⁴¹ à R⁴⁸ sont chacun indépendamment un atome ou un groupe substituant choisi dans le groupe constitué d'un atome d'hydrogène, d'atomes d'halogène, d'un groupe cyano, de groupes alkyle en C1-10, de groupes aryle en C6-10, de groupes amino éventuellement substitués par un groupe alkyle en C1-10, de groupes alcoxy en C1-10 et de groupes silyle ; une paire de R⁴¹ et R⁴², une paire de R⁴² et R⁴³, une paire de R⁴³ et R⁴⁴, une paire de R⁴⁴ et R⁴⁵, une paire de R⁴⁵ et R⁴⁶, une paire de R⁴⁶ et R⁴⁷, ou une paire de R⁴⁷ et R⁴⁸ peut être liée ensemble pour former un cycle condensé ; et L est un ligand bidenté choisi parmi les formules (2-2) à (2-4) ci-dessous : 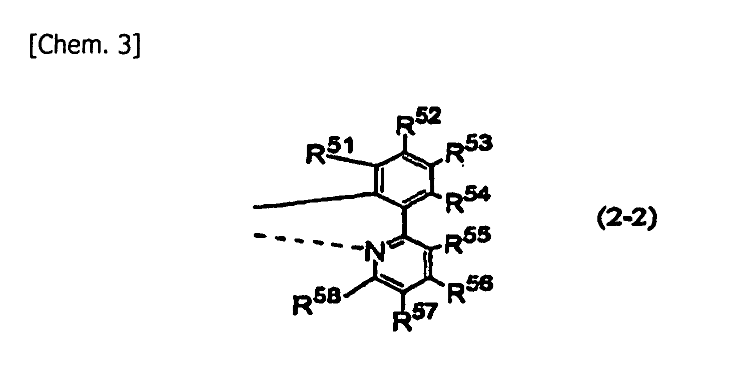 dans laquelle au moins un de R⁵¹ à R⁵⁸ est un groupe substituant présentant un groupe fonctionnel polymérisable, et ceux de R⁵¹ à R⁵⁸ qui ne sont pas des groupes substituants présentant un groupe fonctionnel polymérisable sont chacun indépendamment un atome ou un groupe substituant choisi dans le groupe constitué d'un atome d'hydrogène, d'atomes d'halogène, d'un groupe cyano, de groupes alkyle en C1-10, de groupes aryle en C6-10, de groupes amino éventuellement substitués avec un groupe alkyle en C1-10, de groupes alcoxy en C1-10 et de groupes silyle ; et une paire de R⁵¹ et R⁵², une paire de R⁵² et R⁵³, une paire de R⁵³ et R⁵⁴, une paire de R⁵⁴ et R⁵⁵, une paire de R⁵⁵ et R⁵⁶, une paire de R⁵⁶ et R⁵⁷, ou une paire de R⁵⁷ et R⁵⁸ peut être liée ensemble pour former un cycle condensé ; 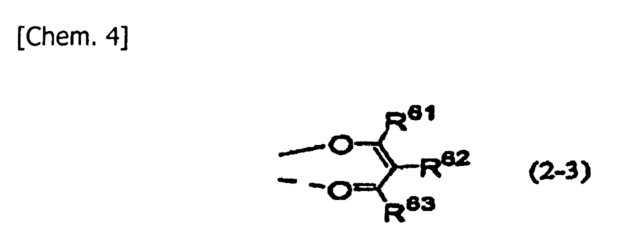 dans laquelle au moins un de R⁶¹ à R⁶³ est un groupe substituant présentant un groupe fonctionnel polymérisable, et ceux de R⁶¹ à R⁶³ qui ne sont pas des groupes substituants présentant un groupe fonctionnel polymérisable sont chacun indépendamment un atome ou un groupe substituant choisi dans le groupe constitué d'un atome d'hydrogène, d'un groupe cyano, de groupes alkyle en C1-10, de groupes aryle en C6-10, de groupes amino éventuellement substitués avec un groupe alkyle en C1-10, de groupes alcoxy en C1-10 et de groupes silyle ; et une paire de R⁶¹ et R⁶² ou une paire de R⁶² et R³³ peut être liée ensemble pour former un cycle condensé ; 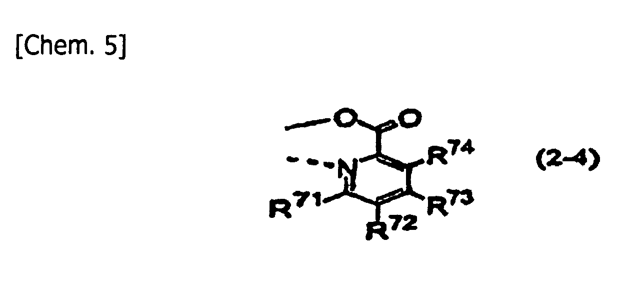 dans laquelle au moins un de R⁷¹ à R⁷⁴ est un groupe substituant présentant un groupe fonctionnel polymérisable, et ceux de R⁷¹ à R⁷⁴ qui ne sont pas des groupes substituants présentant un groupe fonctionnel polymérisable sont chacun indépendamment un atome ou un groupe substituant choisi dans le groupe constitué d'un atome d'hydrogène, d'atomes d'halogène, d'un groupe cyano, de groupes alkyle en C1-10, de groupes aryle en C6-10, de groupes amino éventuellement substitués avec un groupe alkyle en C1-10, de groupes alcoxy en C1-10 et de groupes silyle ; et une paire de R⁷¹ et R⁷² une paire de R⁷² et R⁷³, ou une paire de R⁷³ et R⁷⁴ peut être liée ensemble pour former un cycle condensé.

5. Dispositif organique émettant de la lumière selon la revendication 1, dans lequel la couche émettant de la lumière comprend de plus un composé phosphorescent.

6. Dispositif organique émettant de la lumière selon la revendication 1, dans lequel la couche émettant de la lumière comprend de plus un composé phosphorescent et le composé polymère comprend de plus des unités structurelles d'un composé polymérisable de transport d'électrons (C).

7. Dispositif organique émettant de la lumière selon la revendication 3 ou 6, dans lequel le composé polymérisable de transport d'électrons (C) est un dérivé d'oxadiazole ou un dérivé de triarylborane.

8. Source de lumière émettant en surface dans laquelle on utilise le dispositif organique émettant de la lumière décrit dans l'une quelconque des revendications 1 à 7.

9. Affichage d'image dans lequel on utilise le dispositif organique émettant de la lumière décrit dans l'une quelconque des revendications 1 à 7.
